# EUROPÄISCHE PATENTANMELDUNG

(11) **EP 3 232 545 A1**
(43) Veröffentlichungstag der Anmeldung: **18.10.2017**
(21) Anmeldenummer: 17163483.5
(22) Anmeldetag: 29.03.2017
(51) Int. Cl.: H02K 7/116, H02K 11/22, H02K 29/10

(54) **ELEKTROMOTOR**

(30) Priorität: 11.04.2016 DE 102016106581
(71) Anmelder: Bühler Motor GmbH, 90459 Nürnberg (DE)
(72) Erfinder: Brod, Sabrina, 90641 Nürnberg (DE)

(57) **Zusammenfassung**

Die Erfindung betrifft einen Elektromotor (1) mit einer Motorwelle (4), einem Motorritzel (2) und einem Abtastelement (3) für einen optischen Drehgeber (7), das wenigstens eine Aussparung (5) zum Durchlass eines Lichtstrahls (11) des optischen Drehgebers (7) aufweist, wobei das Motorritzel (2) und das Abtastelement (3) einstückig miteinander ausgebildet und drehfest mit der Motorwelle (4) gekoppelt sind. Die Erfindung zeichnet sich ferner dadurch aus, dass das Abtastelement (3) trommelförmig ausgebildet und koaxial zum Motorritzel (2) ausgerichtet ist, wobei die Aussparung (5) so im Abtastelement (3) ausgebildet ist, dass der Lichtstrahl (11) des Drehgebers (7) radial zu einer Rotationsachse des Abtastelements (3) passieren kann. Die Erfindung betrifft ferner ein medizinisches Gerät mit einem solchen Elektromotor.

## Beschreibung

Die Erfindung betrifft einen Elektromotor gemäß dem Oberbegriff des Patentanspruchs 1. Ferner betrifft die Erfindung ein medizinisches Gerät, insbesondere eine Spritzenpumpe oder Infusionspumpe, mit einem derartigen Elektromotor. Ein Elektromotor der eingangs genannten Art ist beispielsweise aus WO 2013/160351 A2 bekannt.

Der bekannte Elektromotor weist eine elektromechanische Antriebseinheit auf, durch die sich eine Motorwelle erstreckt. Die Motorwelle ragt über ein Gehäuse des Elektromotors hinaus und ist fest mit einem Motorritzel verbunden. Zwischen dem Motorritzel und dem Motorgehäuse ist ein Abtastelement in Form einer mit Aussparungen versehenen Scheibe angeordnet. Das Abtastelement weist einen Querschnittsdurchmesser auf, der größer als der Querschnittsdurchmesser des Motorritzels ist, und ist mit einem optischen Drehgeber wirkverbunden, der radial außerhalb des Abtastelements angeordnet ist. Der Drehgeber bildet eine Lichtschranke, deren Lichtstrahl bei Drehung des Abtastelements temporär abgeschattet wird. Die Aussparungen im Abtastelement erlauben einen Durchlass des Lichtstrahls.

Nachteilig bei dem vorbekannten Elektromotor ist der relativ große radiale Bauraum, der durch die Anordnung des Abtastelements und des Drehgebers benötigt wird. So sind beispielsweise bei medizinischen Spritzenpumpen kleine Dimensionen der Antriebseinheit gefordert, um die Spritzenpumpe insgesamt kompakt gestalten zu können.

Die Aufgabe der Erfindung besteht darin, einen Elektromotor anzugeben, der einen möglichst kleinen radialen Bauraum aufweist und einfach sowie kostengünstig herstellbar ist. Ferner ist es Aufgabe der Erfindung, ein medizinisches Gerät, insbesondere eine Spritzenpumpe oder Infusionspumpe, mit einem solchen Elektromotor anzugeben.

Erfindungsgemäß wird diese Aufgabe im Hinblick auf den Elektromotor durch den Gegenstand des Patentanspruchs 1 und im Hinblick auf das medizinische Gerät durch den Gegenstand des Patentanspruchs 15 gelöst.

So beruht die Erfindung auf dem Gedanken, eine Elektromotor mit einer Motorwelle, einem Motorritzel und einem Abtastelement für einen optischen Drehgeber vorzuschlagen, wobei das Abtastelement wenigstens eine Aussparung zum Durchlass eines Lichtstrahls des optischen Drehgebers aufweist. Das Motorritzel und das Abtastelement sind einstückig miteinander ausgebildet und drehfest mit der Motorwelle gekoppelt. Das Abtastelement ist trommelförmig ausgebildet und koaxial zum Motorritzel ausgerichtet. Dabei ist die Aussparung im Abtastelement so ausgebildet, dass der Lichtstrahl des Drehgebers radial zu einer Rotationsachse des Abtastelements passieren kann.

Durch die trommelförmige Ausbildung des Abtastelements wird es ermöglicht, den Drehgeber axial zum Motorritzel anzuordnen. Damit wird der Bauraum in radialer Richtung reduziert. Die axiale Anordnung des Drehgebers wird auch durch die Anordnung der Aussparung ermöglicht, die so im Abtastelement ausgebildet ist, dass der Lichtstrahl des Drehgebers radial durch die Aussparung passieren kann. Die Trommelform des Abtastelements ist überdies einfach herstellbar und damit kostengünstig.

Vorzugsweise ist das Motorritzel zwischen dem Abtastelement und einem Motorgehäuse angeordnet. Insbesondere ist bei der Erfindung bevorzugt vorgesehen, dass sich das Abtastelement längsaxial über die Motorwelle hinaus erstreckt. Mit anderen Worten kann das Abtastelement stirnseitig am Motorritzel angeordnet sein. Die stirnseitige Anordnung des Abtastelements ermöglicht es, den Drehgeber in Längsrichtung der Motorwelle anzuordnen, um den radialen Bauraum zu reduzieren. Dabei kann der Drehgeber unmittelbar an das Abtastelement gelangen, so dass insgesamt die Herstellung des Elektromotors einfach und kostengünstig ist. Gleichzeitig wird durch die stirnseitige Anordnung des Abtastelements insgesamt eine kompakte Bauweise des Elektromotors ermöglicht.

Bei der Erfindung ist bevorzugt vorgesehen, dass die Aussparung in dem Abtastelement durch zwei Materialsegmente des Abtastelements zum Abschatten des Lichtstrahls des Drehgebers begrenzt ist. Die Materialsegmente bilden gemeinsam mit der Aussparung im Allgemeinen eine Abfolge von abschattenden Bereichen und durchlässigen Bereichen, so dass bei der Rotation des Abtastelements der Lichtstrahl des Drehgebers zeitweise unterbrochen und zeitweise durchgelassen wird. Dadurch entsteht im Drehgeber ein Signal, insbesondere ein Rechtecksignal, wobei die Frequenz der Lichtabschattung und Lichtdurchlässigkeit Aufschluss über die Drehgeschwindigkeit des Elektromotors gibt.

Bevorzugt ist vorgesehen, dass sich die zwei Materialsegmente parallel zu einer Rotationsachse des Abtastelements erstrecken und zwei Aussparungen begrenzen. Die Aussparungen können insbesondere durch eine radial vollständig durch das Abtastelement verlaufende Nut gebildet sein. Eine solche Gestaltung des Abtastelements ist besonders einfach herstellbar. Insbesondere ist eine solche Gestaltung für kleine Durchmesser des Abtastelements bevorzugt, bei welchen der Raum für eine höhere Anzahl von Aussparungen nicht ausreicht. Durch die radial vollständig durch das Abtastelement verlaufende Nut werden im Wesentlichen zwei Aussparungen gebildet, die in einem Winkel von 180 Grad gegenüberliegend angeordnet sind. Jedenfalls kann der Lichtstrahl des Drehgebers vollständig durch die Nut hindurchtreten, so dass die Nut bei einer vollständigen Umdrehung des Abtastelements zweimal den Lichtstrahl passieren lässt. Die die Nut begrenzenden Materialsegmente schatten den Lichtstrahl ab.

Insbesondere kann vorgesehen sein, dass jedes der beiden Materialsegmente eine zylindersegmentförmige Außenfläche und eine ebene Innenfläche aufweist. Die Innenflächen der Materialsegmente sind vorzugsweise parallel zueinander angeordnet und begrenzen die Nut. Mit anderen Worten bilden die Innenflächen der Materialsegmente die Nutseitenwände. Indem die Materialsegmente eine vollflächige, insbesondere teilkreisförmige, Querschnittskontur aufweisen, ist die Stabilität der Materialsegmente vergleichsweise hoch, auch wenn der Gesamtquerschnittsdurchmesser des Abtastelements sehr klein ist. Insgesamt wird so selbst bei kleinen Strukturen eine hohe Stabilität gewährleistet.

Bei einer bevorzugten Ausführungsform des Elektromotors ist vorgesehen, dass das Abtastelement hohlzylinderförmig ausgebildet ist. Dabei können die Materialsegmente eine einheitliche Wandstärke aufweisen. Insbesondere können die Materialsegmente wenigstens zwei, insbesondere wenigstens drei, insbesondere wenigstens vier, Aussparungen begrenzen. Durch eine höhere Anzahl von Aussparungen wird die Genauigkeit bei der Ermittlung der Drehzahl des Elektromotors und der aktuellen Drehstellung des Elektromotors erhöht. Dies ist in vielen Anwendungen bevorzugt. Durch die hohlzylinderförmige Gestaltung des Abtastelements wird außerdem die Anordnung des Drehgebers verbessert. Insbesondere kann der innere, zylinderförmige Hohlraum im Abtastelement genutzt werden, um den Drehgeber zumindest teilweise in das Abtastelement eingreifen zu lassen. Dies trägt weiter zur Reduktion des radialen Bauraums bei.

Für alle Ausführungsformen der Erfindung gilt, dass das Motorritzel und das Abtastelement vorzugsweise als ein einziges, insbesondere einstückiges, Spritzgussteil ausgebildet sind. Dabei können das Motorritzel und das Abtastelement als einziges Kunststoffspritzgussteil oder als Metallpulverspritzgussteil, insbesondere durch metal-injected moulding, hergestellt sein. Diese Herstellungsweise vereinfacht weiter die Serienproduktion des Elektromotors und trägt zur Reduktion der Herstellungskosten bei. Überdies wird der Werkzeugeinsatz bei der Herstellung reduziert, da beim Spritzgießen gleichzeitig auch die Aussparung im Abtastelement hergestellt werden kann.

Bei einer bevorzugten Variante des erfindungsgemäßen Elektromotors ist ein Drehgeber mit einer Lichtquelle und einem Lichtempfänger vorgesehen. Dabei kann das Abtastelement so zwischen der Lichtquelle und dem Lichtempfänger angeordnet sein, dass ein direkter Lichtstrahl zwischen der Lichtquelle und dem Lichtempfänger durch die Aussparung freigebbar und/oder durch die Materialsegmente abschattbar ist. Der Drehgeber kann im Wesentlichen eine Lichtschranke bilden, wodurch sich eine hohe Genauigkeit bei der Erkennung von Drehzahl und Drehposition des Elektromotors ergibt. Außerdem ist eine Lichtschranke kaum störanfällig, so dass eine hohe Betriebssicherheit des Elektromotors gewährleistet ist.

Der Lichtstrahl des Drehgebers ist vorzugsweise radial zur Längsachse des Abtastelements ausgerichtet. Die radiale Ausrichtung des Lichtstrahls folgt aus der axial zur Motorwelle ausgerichteten Positionierung des Drehgebers, wobei diese Positionierung des Drehgebers zu einer Reduktion des radialen Bauraums des Elektromotors führt.

Bei einer bevorzugten Variante ist vorgesehen, dass die Lichtquelle und der Lichtempfänger radial außerhalb des Abtastelements einander gegenüberliegend angeordnet sind. Mit anderen Worten ist der Drehgeber so angeordnet, dass das Abtastelement vollständig zwischen Lichtquelle und Lichtempfänger hineinragt. Insbesondere kann der Drehgeber koaxial zum Abtastelement ausgerichtet sein. Eine solche Anordnung des Drehgebers ist besonders einfach realisierbar und erhöht die Wartungsfreundlichkeit, da der Drehgeber einfach seitlich entnommen und gewechselt werden kann.

Bei einer weiteren bevorzugten Ausgestaltung des erfindungsgemäßen Elektromotors sind die Lichtquelle radial innerhalb des Abtastelements und der Lichtempfänger radial außerhalb des Abtastelements angeordnet. Alternativ können der Lichtempfänger radial innerhalb des Abtastelements und die Lichtquelle radial außerhalb des Abtastelements angeordnet sein. Die vorgenannten Anordnungsmöglichkeiten des Drehgebers sind insbesondere vorgesehen, wenn das Abtastelement hohlzylinderförmig ausgebildet ist. Dann kann der Drehgeber so angeordnet sein, dass ein Lichtschrankenelement, insbesondere die Lichtquelle oder der Lichtempfänger, innerhalb des Abtastelements und ein anderes Lichtschrankenelement, insbesondere der Lichtempfänger oder die Lichtquelle, außerhalb des Abtastelements angeordnet ist. Insgesamt kann so eine besonders kompakte Gestaltung des Elektromotors erreicht werden.

Vorzugsweise sind die Lichtquelle und der Lichtempfänger auf einer gemeinsamen Sensorleiterplatte befestigt. Die Sensorleiterplatte ist vorzugsweise axial vom Abtastelement beabstandet angeordnet. Die Verwendung einer gemeinsamen Sensorleiterplatte erleichtert die Herstellung des Elektromotors und führt zu einer kompakten Bauweise. Insbesondere wird im Unterschied zu aus dem Stand der Technik bekannten Elektromotoren vermieden, dass die Sensorleiterplatte seitlich zum Elektromotor angeordnet ist, was den radialen Bauraum erhöhen würde.

Durch die axiale Ausrichtung der Sensorleiterplatte wird vielmehr der radiale Bauraum reduziert.

Bei einer weiteren bevorzugten Ausgestaltung des Elektromotors sind wenigstens zwei Drehgeber vorgesehen, die jeweils eine Lichtschranke bilden. Dabei können die Lichtschranken um einen Winkel um die Längsachse des Abtastelements zueinander versetzt sein, der ungleich 180 Grad beträgt. Insbesondere kann der Winkel auch ungleich 90 Grad betragen. Die Verwendung von wenigstens zwei Drehgebern führt zu einer vorteilhaften Redundanz, so dass bei Ausfall eines Drehgebers weiterhin ein Betrieb des Elektromotors ermöglicht ist. Dies ist insbesondere bei medizinischen Anwendungen bevorzugt. Durch die Anordnung der Drehgeber derart, dass deren Lichtschranken unter einem spitzen Winkel zueinander angeordnet sind, wird außerdem die Erkennungsgenauigkeit erheblich verbessert. Insbesondere kann durch die zwei Drehgeber, vorzugsweise mit spitzwinklig zueinander angeordneten Lichtschranken, eine Drehrichtungserkennung ermöglicht werden. Damit wird nicht nur die Drehgeschwindigkeit und die derzeitige Drehposition des Elektromotors erfasst, sondern auch die Richtung, in welche sich der Elektromotor, insbesondere die Motorwelle, dreht.

Die eingangs genannte Aufgabe der Erfindung wird insbesondere auch durch ein medizinisches Gerät, insbesondere eine Spritzenpumpe oder eine Infusionspumpe, gelöst, die einen zuvor beschriebenen Elektromotor aufweist. Besonders bevorzugt ist der Einsatz des Elektromotors in einer Spritzenpumpe, wobei die Spritzenpumpe ferner ein Stirnradgetriebe aufweisen kann, das mit dem Motorritzel des Elektromotors verbunden ist. Das Stirnradgetriebe kann mit einer Gewindespindel gekoppelt sein, die auf einen Spritzenkolben einer Spritze wirkt, welche in die Spritzenpumpe eingespannt ist. Auf diese Weise können mit hoher Genauigkeit kleine Mengen eines Medikaments über einen längeren Zeitraum kontinuierlich verabreicht bzw. infundiert werden.

Die Erfindung wird im Folgenden anhand von Ausführungsbeispielen unter Bezugnahme auf die beigefügten, schematischen Zeichnungen näher erläutert. Darin zeigen:
- Fig. 1: eine perspektivische Ansicht eines erfindungsgemäßen Elektromotors nach einem ersten bevorzugten Ausführungsbeispiel;
- Fig. 2: eine perspektivische Ansicht des Elektromotors gemäß Fig. 1 mit einem Drehgeber;
- Fig. 3: eine Seitenansicht des Elektromotors gemäß Fig. 2;
- Fig. 4: eine perspektivische Ansicht eines Elektromotors mit Drehgeber nach einem zweiten bevorzugten Ausführungsbeispiel mit alternativer Gestaltung des Abtastelements;
- Fig. 5: eine Seitenansicht des Elektromotors gemäß Fig. 4;
- Fig. 6: eine Querschnittsansicht durch einen Teil einer Spritzenpumpe mit dem Elektromotor gemäß Fig. 4;
- Fig. 7: eine Querschnittsansicht durch einen Teil eines Elektromotors nach einem dritten bevorzugten Ausführungsbeispiel, wobei das Abtastelemente vollständig in einen Drehgeber hineinragt; und
- Fig. 8: eine Querschnittsansicht eines Teils eines erfindungsgemäßen Elektromotors nach einem weiteren bevorzugten Ausführungsbeispiel mit alternativer Gestaltung des Motorritzels und Abtastelements.

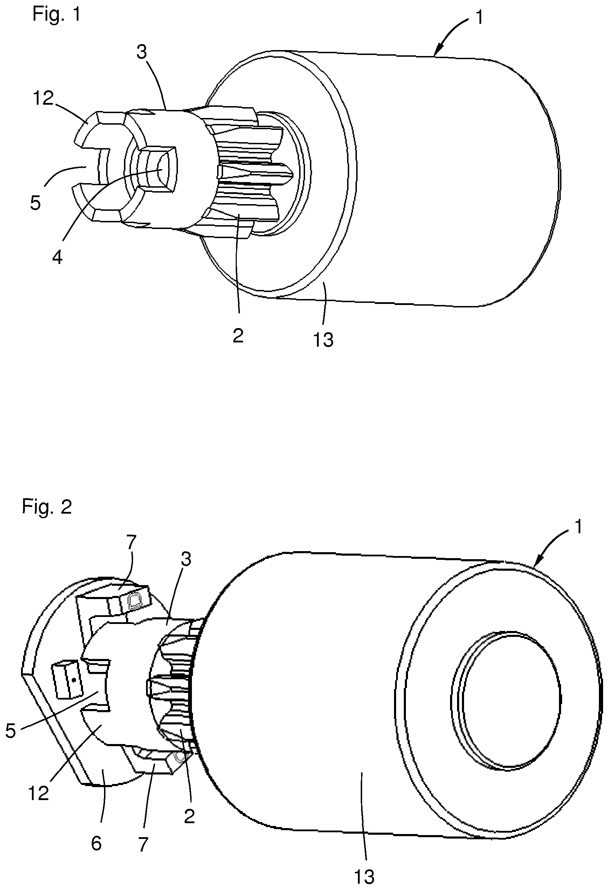

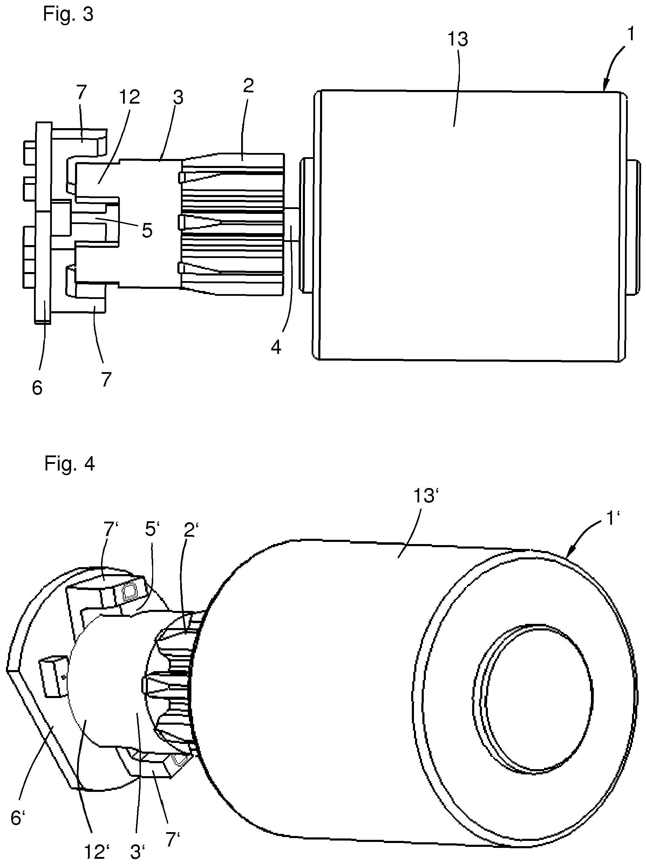

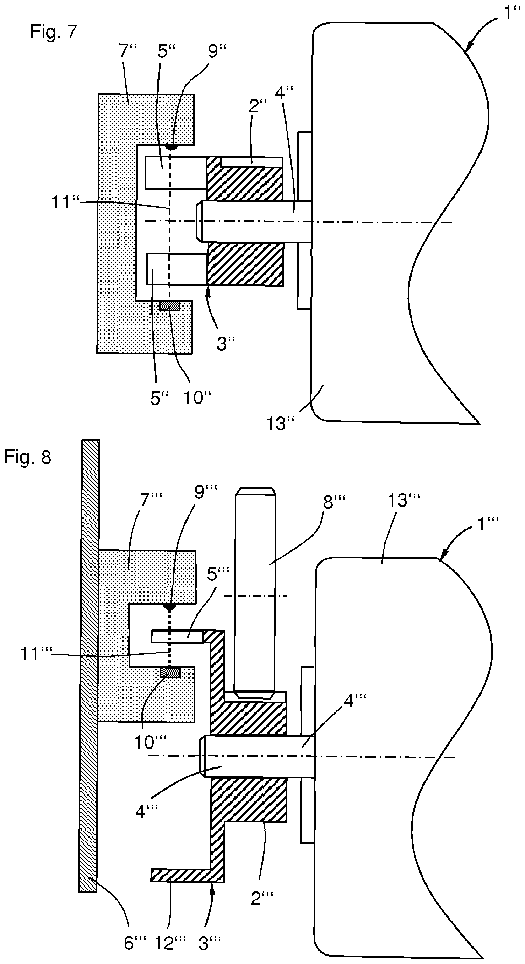

Die beigefügten Zeichnungen zeigen insgesamt vier unterschiedliche Ausführungsbeispiele der Erfindung. Dabei sind die Bezugszeichen zu den einzelnen Ausführungsbeispielen durch eine unterschiedliche Anzahl von Hochindizes gekennzeichnet. Die Fig. 1 bis 3 zeigen ein erstes Ausführungsbeispiel, die Fig. 4 bis 6 ein zweites Ausführungsbeispiel, die Fig. 7 ein drittes Ausführungsbeispiel und die Fig. 8 ein viertes Ausführungsbeispiel.

Allen Ausführungsbeispielen gemeinsam ist ein Elektromotor 1, der ein Motorgehäuse 13 aufweist. Aus dem Motorgehäuse ragt längsaxial eine Motorwelle 4 hervor, auf welcher ein Motorritzel 2 sitzt. Das Motorritzel 2 ist einstückig mit einem Abtastelement 3 verbunden, wobei das Abtastelement 3 trommelförmig ausgebildet ist. Insbesondere weist das Abtastelement 3 eine zylinderförmige Außenumfangsfläche auf. Das Abtastelement 3 ist bei allen Ausführungsbeispielen koaxial zum Motorritzel 2 ausgerichtet. Insbesondere sind das Motorritzel und das Abtastelement einstückig miteinander ausgebildet, vorzugsweise als einziges Spritzgussteil.

Das Abtastelement weist wenigstens eine Aussparung 5 auf, die einen Durchlass für einen Lichtstrahl 11 eines Drehgebers 7 bildet. Bei dem ersten Ausführungsbeispiel ist in Fig. 1 gut erkennbar, dass das Abtastelement 3 im Wesentlichen hohlzylinderförmig ausgebildet ist. Insbesondere weist das Abtastelement 3 Materialsegmente 12 auf, die jeweils Aussparungen 5 begrenzen. Die Materialsegmente 12 sind bogenförmig gekrümmt. Konkret folgen die Materialsegmente 12 der Hohlzylinderform des Abtastelements 3. Die Materialsegmente 12 weisen eine einheitliche Wandstärke auf.

An das Abtastelement 3 schließt sich einstückig das Motorritzel 2 an, das drehfest mit der Motorwelle 4 verbunden ist. Die Motorwelle 4 endet vorzugsweise im Bereich einer Bodenfläche des Abtastelements 3. Insbesondere ragt das Abtastelement 3 längsaxial über die Motorwelle 4 hinaus. Insoweit ist das Motorritzel 2 zwischen dem Abtastelement 3 und dem Motorgehäuse 13 angeordnet.

Das Abtastelement 3 weist bei dem ersten Ausführungsbeispiel vier Aussparungen auf, die jeweils in einem Winkeln von 90 Grad zueinander versetzt angeordnet sind. Die Aussparungen 5 weisen eine einheitliche Breite auf. Ferner sind die Aussparungen 5 einheitlich zueinander beabstandet. Mit anderen Worten weisen auch die Materialsegmente 2 eine einheitliche Breite auf. Auf diese Weise sind die Aussparungen 5 so angeordnet, dass jeweils zwei Aussparungen 5 radial gegenüberliegend angeordnet sind. Dabei bezieht sich die Radialität auf die Längsachse der Motorwelle 4, also die Rotationsachse des Elektromotors 1.

Wie in Fig. 2 gut erkennbar ist, weist der Elektromotor 1 zudem einen Encoder mit einer Sensorleiterplatte 6 auf, wobei auf der Sensorleiterplatte 6 zwei Drehgeber 7 angeordnet sind. Die Drehgeber 7 bilden jeweils eine Lichtschranke. Dazu weist jeder Drehgeber 7 eine Lichtquelle 9 und einen Lichtempfänger 10 auf. Zwischen der Lichtquelle 9 und dem Lichtempfänger 10 ist ein Lichtstrahl 11 aktivierbar, der bei Drehung des Elektromotors 1 durch das Abtastelement 3 temporär unterbrochen wird. Insbesondere sorgen die Materialsegmente 12 jeweils für eine Unterbrechung des Lichtstrahls 11, wogegen die Aussparungen 5 den Lichtstrahl 11 passieren lassen.

Bei dem ersten Ausführungsbeispiel ist vorgesehen, dass die Drehgeber 7 so angeordnet sind, dass jeweils ein Lichtschrankenelement, d.h. die Lichtquelle 9 oder der Lichtempfänger 10, in das Abtastelement 3 eingreift. Insbesondere ist ein Lichtschrankenelement radial innerhalb des Abtastelements 3 angeordnet, wogegen ein anderes Lichtschrankenelement radial außerhalb des Abtastelements 3 angeordnet ist. Beide Drehgeber 7 sind auf der gemeinsamen Sensorleiterplatte 6 angeordnet, die axial von dem Abtastelement 3 beabstandet positioniert ist. Die Sensorleiterplatte 6 ist im Wesentlichen koaxial zum Motorgehäuse 3 ausgerichtet.

Die Anordnung der Drehgeber 7 bezüglich des Abtastelements 3 ist gut in Fig. 3 erkennbar. Dabei ist auch zu sehen, dass bei dem ersten Ausführungsbeispiel das Abtastelement 3 einen Querschnittsdurchmesser aufweist, der kleiner als der Querschnittsdurchmesser des Motorritzels 2 ist. Somit ist eine besonders kompakte Bauweise erreicht, insbesondere im Hinblick auf den radialen Bauraum.

In Fig. 2 ist außerdem gut erkennbar, dass die beiden Lichtschranken, die durch die zwei Drehgeber 7 gebildet sind, zueinander winklig ausgerichtet sind. Die Lichtschranken sind also so positioniert, dass deren Lichtstrahlen 11 zueinander einen Winkel einnehmen, der ungleich 180 Grad und ungleich 90 Grad ist. Die Lichtschranken sind insoweit spitzwinklig zueinander angeordnet. Dadurch kann eine Drehrichtungserkennung realisiert werden.

In den Fig. 4 bis 6 ist ein zweites Ausführungsbeispiel der Erfindung gezeigt, das sich von dem ersten Ausführungsbeispiel durch die Gestaltung des Abtastelements 3' unterscheidet. Die übrigen Komponenten sind im Wesentlichen identisch ausgebildet. So weist der Elektromotor 1' bei dem zweiten Ausführungsbeispiel ebenfalls ein Motorgehäuse 13' auf, durch welches sich eine Motorwelle 4' erstreckt. Die Motorwelle 4' ist drehfest mit einem Motorritzel 2' verbunden. Stirnseitig schließt sich an das Motorritzel 2' das Abtastelement 3' an.

Das Abtastelement 3' ist hohlzylinderförmig ausgebildet und weist zwei Materialsegmente 12' auf, die zwei Aussparungen 5' begrenzen. Die Materialsegmente 12' weisen eine einheitliche Wandstärke auf. Vorzugsweise weisen die Materialsegmente 12' eine einheitliche Breite auf, so dass auch die Aussparungen 5' eine einheitliche Breite umfassen. Die Aussparungen 5' sind radial gegenüberliegend zueinander angeordnet.

Im Unterschied zu dem ersten Ausführungsbeispiel ist bei dem zweiten Ausführungsbeispiel vorgesehen, dass das Abtastelement 3' lediglich zwei Aussparungen 5' aufweist. Diese Aussparungen 5' lassen Lichtstrahlen zweier Drehgeber 7' passieren, die auf einer gemeinsamen Sensorleiterplatte 6' angeordnet sind. Die gemeinsame Sensorleiterplatte 6' ist axial beabstandet zum Abtastelement 3' angeordnet.

Die Drehgeber 7' weisen jeweils eine Lichtschrankenelement auf, das radial innerhalb des Abtastelements 3' angeordnet ist. Jedes der Drehgeber 7' weist außerdem ein weiteres Lichtschrankenelement auf, das radial außerhalb des Abtastelements 3' angeordnet ist. Die Lichtstrahlen 11 der Drehgeber 7' sind spitzwinklig zueinander ausgerichtet.

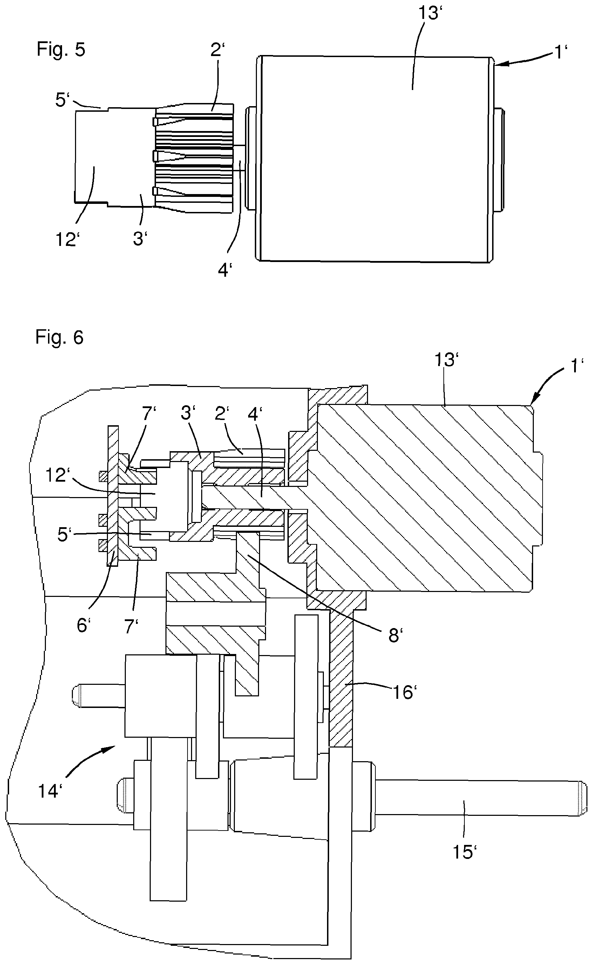

In Fig. 6 ist beispielhaft anhand des zweiten Ausführungsbeispiels eine Anordnung des Elektromotors 1 innerhalb einer Spritzenpumpe gezeigt. Eine solche Anordnung lässt sich auch allen weiteren Ausführungsbeispielen realisieren und ist nicht auf das zweite Ausführungsbeispiel eingeschränkt.

Im Allgemeinen weist die Spritzenpumpe ein Antriebsgehäuse 16' auf, das den Elektromotor 1', insbesondere das Motorgehäuse 13', aufnimmt. Im Antriebsgehäuse 16' ist außerdem ein Stirnradgetriebe 14' gelagert, welches über ein Stirnrad 8' mit dem Motorritzel 2' des Elektromotors 1' gekoppelt ist. Das Stirnradgetriebe 14' wirkt auf eine Gewindespindel 15'. Die Gewindespindel 15' ist mit einer Spritzenkolbenaufnahme verbunden. So kann eine Bewegung der Gewindespindel 15' auf einen Spritzenkolben übertragen werden.

Die Spritzenpumpe funktioniert im Wesentlichen wie folgt. Durch den Elektromotor 1' wird die Motorwelle 4' in Rotation versetzt. Die Motorwelle 4' ist mit dem Motorritzel 2' drehfest verbunden. So wird die Rotation der Motorwelle 4' unmittelbar auf das Motorritzel 2' übertragen, welches folglich mit derselben Geschwindigkeit wie die Motorwelle 4' rotiert. Die Drehgeschwindigkeit und Drehrichtung wird dabei über die Drehgeber 7' des Encoders ermittelt und ggf. anhand voreingestellter Werte angepasst.

Das Motorritzel 2' kämmt mit dem Stirnrad 8', dessen Drehbewegung auf das Stirnradgetriebe 14' übertragen wird. Das Stirnradgetriebe 14' überträgt die Drehbewegung auf die Gewindespindel 15', die die Rotationsbewegung in eine lineare Bewegung umsetzt. Über das Übersetzungsverhältnis des Stirnradgetriebes 14' wird erreicht, dass die Gewindespindel 15' mit konstanter, insbesondere sehr langsamer, Geschwindigkeit eine längsaxiale Bewegung vollzieht. So können Medikamente aus einer Spritze kontinuierlich mit gleichbleibendem Fluidfluss abgegeben werden.

In Fig. 7 ist ein drittes Ausführungsbeispiel der Erfindung gezeigt. Im Wesentlichen ist ausschnittsweise ein Elektromotor 1" dargestellt, der eine Motorwelle 4" umfasst. Mit der Motorwelle 4" ist ein Motorritzel 2" drehfest verbunden. Über die Motorwelle 4" hinaus ragt ein Abtastelement 3", welches einstückig mit dem Motorritzel 2" ausgebildet ist. Das Abtastelement 3" umfasst mehrere Aussparungen 5", die einen Lichtstrahl 11" eines Drehgebers 7" passieren lassen. Der Drehgeber 7" umfasst dazu eine Lichtquelle 9" und einen Lichtempfänger 10", die radial bezogen auf die Motorwelle 4" zueinander gegenüberliegend angeordnet sind.

Bei dem dritten Ausführungsbeispiel gemäß Fig. 7 ist vorgesehen, dass der Drehgeber 7" vollständig radial außerhalb des Abtastelements 3" angeordnet ist. Insbesondere weist der Drehgeber 7" zwei Lichtschrankenelemente, insbesondere die Lichtquelle 9" und den Lichtempfänger 10", auf, die jeweils radial außerhalb des Abtastelements 2" positioniert sind. Der Lichtstrahl 11" verläuft somit vollständig das Abtastelement 3". Dazu ist vorgesehen, dass das Abtastelement 3" wenigstens zwei Aussparungen 5" aufweist, die radial gegenüberliegend angeordnet sind.

Ein viertes Ausführungsbeispiel der Erfindung ist in Fig. 8 dargestellt. Darin ist ein Elektromotor 1'" gezeigt, der ein Motorgehäuse 13'" aufweist. Aus dem Motorgehäuse 13'" ragt koaxial eine Motorwelle 4'" vor, die drehfest mit einem Motorritzel 2'" verbunden ist. Das Motorritzel 2'" kämmt mit einem Stirnrad 8"'.

An das Motorritzel 2'" schließt sich längsaxial und einstückig ein Abtastelement 3'" an. Das Abtastelement 3'" ist im Wesentlichen hohlzylinderförmig ausgebildet. Insbesondere weist das Abtastelement 3'" ein oder mehrere Materialsegmente 12'" auf, die wenigstens eine Aussparung 5'" begrenzen.

In axialer Richtung beabstandet zum Motorgehäuse 13'" angeordnet ist ein Encoder mit einer Sensorleiterplatte 6'" und wenigstens einem Drehgeber 7'", der mit der Sensorleiterplatte 6"' fest verbunden ist. Der Drehgeber 7"' umfasst eine Lichtquelle 9'" und einen Lichtempfänger 10"'. Die Lichtquelle 9'" und der Lichtempfänger 10'" sind zueinander radial gegenüberliegend angeordnet. Zwischen der Lichtquelle 9'" und dem Lichtempfänger 10'" ist ein Lichtstrahl 11"' aktivierbar.

Wie in Fig. 8 gut erkennbar ist, weist das Abtastelement 3'" einen Querschnittsdurchmesser auf, der deutlich größer als der Querschnittsdurchmesser des Motorritzels 2'" ist. So kann auch bei einem kleinen Motorritzel 2'" ein Drehgeber 7'" so angeordnet werden, dass der Lichtempfänger 10'" radial innerhalb des Abtastelements 3'" angeordnet ist, wogegen die Lichtquelle 9'" radial außerhalb des Abtastelements 3'" positioniert ist. Insbesondere kann so mehr als ein Drehgeber 7'" vorgesehen sein, um die Genauigkeit der Messung zu verbessern.

Die zuvor beschriebene Erfindung eignet sich generell für besonders kompakte Elektromotoren. Insbesondere ist die Erfindung besonders geeignet für Elektromotoren mit einem Außendurchmesser, insbesondere Gehäuseaußendurchmesser, von 8 mm.

### Bezugszeichenliste

- 1, 1', 1", 1'": Elektromotor
- 2, 2', 2", 2'": Motorritzel
- 3, 3', 3", 3'": Abtastelement
- 4, 4', 4", 4"': Motorwelle
- 5, 5', 5", 5"': Aussparung
- 6, 6', 6", 6'": Sensorleiterplatte
- 7, 7', 7", 7"': Drehgeber
- 8, 8', 8", 8"': Stirnrad
- 9", 9'": Lichtquelle
- 10", 10'": Lichtempfänger
- 11", 11'": Lichtstrahl
- 12, 12', 12", 12'": Materialsegment
- 13, 13', 13", 13'": Motorgehäuse
- 14': Stirnradgetriebe
- 15': Gewindespindel
- 16': Antriebsgehäuse

## Patentansprüche

1. Elektromotor (1) mit einer Motorwelle (4), einem Motorritzel (2) und einem Abtastelement (3) für einen optischen Drehgeber (7), das wenigstens eine Aussparung (5) zum Durchlass eines Lichtstrahls (11) des optischen Drehgebers (7) aufweist, wobei das Motorritzel (2) und das Abtastelement (3) einstückig miteinander ausgebildet und drehfest mit der Motorwelle (4) gekoppelt sind,
**dadurch gekennzeichnet, dass**
das Abtastelement (3) trommelförmig ausgebildet und koaxial zum Motorritzel (2) ausgerichtet ist, wobei die Aussparung (5) so im Abtastelement (3) ausgebildet ist, dass der Lichtstrahl (11) des Drehgebers (7) radial zu einer Rotationsachse des Abtastelements (3) passieren kann.

2. Elektromotor (1) nach Anspruch 1,
**dadurch gekennzeichnet, dass**
das Motorritzel (2) zwischen dem Abtastelement (3) und einem Motorgehäuse (13) angeordnet ist.

3. Elektromotor (1) nach Anspruch 1 oder 2,
**dadurch gekennzeichnet, dass**
sich das Abtastelement (3) längsaxial über die Motorwelle (4) hinaus erstreckt.

4. Elektromotor (1) nach einem der vorhergehenden Ansprüche,
**dadurch gekennzeichnet, dass**
die Aussparung (5) durch zwei Materialsegmente (12, 12') zum Abschatten des Lichtstrahls (11) des Drehgebers (7) begrenzt ist.

5. Elektromotor (1) nach Anspruch 4,
**dadurch gekennzeichnet, dass**
sich die zwei Materialsegmente (12') parallel zu einer Rotationsachse des Abtastelements (3) erstrecken und zwei Aussparungen (5) begrenzen, die durch eine radial vollständig durch das Abtastelement (3) verlaufende Nut gebildet ist.

6. Elektromotor (1) nach Anspruch 5,
**dadurch gekennzeichnet, dass**
jedes der beiden Materialsegmente (12') eine zylindersegmentförmige Außenfläche und eine ebene Innenfläche aufweist, wobei die Innenflächen der Materialsegmente (12') parallel zueinander angeordnet sind und die Nut begrenzen.

7. Elektromotor (1) nach Anspruch 4,
**dadurch gekennzeichnet, dass**
das Abtastelement (3) hohlzylinderförmig ausgebildet ist, wobei die Materialsegmente (12, 12') eine einheitliche Wandstärke aufweisen und wenigstens 2, insbesondere wenigstens 3, insbesondere wenigstens 4, Aussparungen (5) begrenzen.

8. Elektromotor (1) nach einem der vorhergehenden Ansprüche,
**dadurch gekennzeichnet, dass**
das Motorritzel (2) und das Abtastelement (3) als ein einziges, insbesondere einstückiges, Spritzgussteil ausgebildet sind.

9. Elektromotor (1) nach einem der vorhergehenden Ansprüche,
**dadurch gekennzeichnet, dass**
ein Drehgeber (7) mit einer Lichtquelle (9) und einem Lichtempfänger (10) vorgesehen ist, wobei das Abtastelement (3) so zwischen der Lichtquelle (9) und dem Lichtempfänger (10) angeordnet ist, dass ein direkter Lichtstrahl (11) zwischen der Lichtquelle (9) und dem Lichtempfänger (10) durch die Aussparung (5) freigebbar und/oder durch die Materialsegmente (12, 12') abschattbar ist.

10. Elektromotor (1) nach Anspruch 9,
**dadurch gekennzeichnet, dass**
der Lichtstrahl (11) radial zur Längsachse des Abtastelements (3) ausgerichtet ist.

11. Elektromotor (1) nach Anspruch 9 oder 10,
**dadurch gekennzeichnet, dass**
die Lichtquelle (9) und der Lichtempfänger (10) radial außerhalb des Abtastelements (3) einander gegenüberliegend angeordnet sind.

12. Elektromotor (1) nach einem der Ansprüche 9 bis 10,
**dadurch gekennzeichnet, dass**
die Lichtquelle (9) radial innerhalb des Abtastelements (3) und der Lichtempfänger (10) radial außerhalb des Abtastelements (3)
oder
der Lichtempfänger (10) radial innerhalb des Abtastelements (3) und die Lichtquelle (9) radial außerhalb des Abtastelements (3) angeordnet sind.

13. Elektromotor (1) nach einem der Ansprüche 9 bis 12,
**dadurch gekennzeichnet, dass**
die Lichtquelle (9) und der Lichtempfänger (10) auf einer gemeinsamen Sensorleiterplatte (6) befestigt sind, die axial vom Abtastelement (3) beabstandet angeordnet ist.

14. Elektromotor (1) nach einem der Ansprüche 9 bis 13,
**dadurch gekennzeichnet, dass**
wenigstens zwei Drehgeber (7) vorgesehen sind, die jeweils eine Lichtschranke bilden, wobei die Lichtschranken um einen Winkel um die Längsachse des Abtastelements (3) versetzt sind, der ungleich 180 Grad beträgt.

15. Medizinisches Gerät, insbesondere Spritzenpumpe oder Infusionspumpe, mit einem Elektromotor (1) nach einem der vorhergehenden Ansprüche.
